(19) Europäisches Patentamt
European Patent Office
Office européen des brevets

(11) **EP 2 637 710 B1**

(12) **EUROPEAN PATENT SPECIFICATION**

(45) Date of publication and mention
of the grant of the patent:
**05.04.2017 Bulletin 2017/14**

(51) Int Cl.:
***A61L 27/26*** (2006.01)    ***A61L 27/50*** (2006.01)
***A61L 27/52*** (2006.01)

(21) Application number: **11799644.7**

(86) International application number:
**PCT/EP2011/069685**

(22) Date of filing: **08.11.2011**

(87) International publication number:
**WO 2012/062775 (18.05.2012 Gazette 2012/20)**

(54) **HYALURONIC ACID BASED FORMULATIONS**

FORMULIERUNGEN AUF HYALURONSÄUREBASIS

FORMULATIONS À BASE D'ACIDE HYALURONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.11.2010 PCT/IB2010/003008
08.11.2010 PCT/IB2010/002846**

(43) Date of publication of application:
**18.09.2013 Bulletin 2013/38**

(60) Divisional application:
**16193473.2 / 3 138 586**

(73) Proprietor: **Allergan Industrie, SAS
74370 Pringy (FR)**

(72) Inventors:
• **LEBRETON, Pierre
74000 Annecy (FR)**
• **GUETTA, Olivier
74000 Annecy (FR)**

(74) Representative: **Hoffmann Eitle
Patent- und Rechtsanwälte PartmbB
Arabellastraße 30
81925 München (DE)**

(56) References cited:
**WO-A1-2008/068297    US-A1- 2006 194 758
US-A1- 2010 028 437    US-A1- 2010 255 068**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 2 637 710 B1

**Description**

The present invention generally relates to injectable soft tissue fillers and more specifically relates to hyaluronic acid-based dermal and subdermal fillers having improved properties.

Skin is composed of the epidermis and the dermis. Below these layers lies the hypodermis, which is not usually classified as a layer of skin. The hypodermis is also commonly referred to as subcutaneous fat layer, or subcutaneous tissue. The outermost epidermis is made up of stratified squamous epithelium with an underlying basement membrane. It contains no blood vessels, and is nourished by diffusion from the dermis. The main type of cells which make up the epidermis are keratinocytes, with melanocytes and langerhans cells also present. This layer of skin is responsible for keeping water in the body and keeping other harmful chemicals and pathogens out.

The dermis lies below the epidermis and contains a number of structures including blood vessels, nerves, hair follicles, smooth muscle, glands and lymphatic tissue. The dermis (or corium) is typically 3-5 mm thick and is the major component of human skin. It is composed of a network of connective tissue, predominantly collagen fibrils providing support and elastic tissue providing flexibility. The main cell types are fibroblasts, adipocytes (fat storage) and macrophages. The hypodermis lies below the dermis. Its purpose is to attach the skin to underlying bone and muscle as well as supplying it with blood vessels and nerves. It is made up of loose connective tissue and elastin. The main cell types are fibroblasts, macrophages and adipocytes. The hypodermis contains 50% of body fat. Fat serves as padding and insulation for the body. Hyaluronic acid (HA) is a part of the dermis composition and is a major component of the extra cellular matrix.

Facial aging occurs as the result of several factors: inherent changes within the skin, effects of gravity, facial muscles acting on the skin (dynamic lines), soft tissue loss or shift and bone loss and loss of tissue elasticity. The skin ages when the epidermis begins to thin, causing the junction with the dermis to flatten. Collagen decreases as a person ages and the bundles of collagen, which gives the skin turgor, become looser and lose strength. When the skin loses elasticity, it is less able to resist stretching. Coupled with gravity, muscle pull and tissue changes, the skin begin to wrinkle. Water loss and breakdown of bonds between cells also reduces the barrier function of the skin, which can cause the skin's pore size to increases.

As a person ages, the face loses volume, soft tissue, and fat. The appearance of jowls and folds are usually caused by the drooping of facial tissues and folding of areas where the muscles below are attached to the skin. As part of the reduction in soft tissue, the aging face appears more hollow.

More specifically, in various facial areas, such as forehead, eyes, nose, midface and lower face, changes relating to aging have been well documented. In forehead area, the forehead and brow droop over time, which lowers the eyebrows and causes the upper eyelid skin to bunch. Forehead lines appear when one tries to hold the brows and eyelids up to counteract these changes. It is well known that the eyes are often the first facial feature to show signs of aging. Skin changes around the eyes occur earlier than in the rest of the face since the skin is thinner around the eyes. The skin here contains fewer glands and is subjected to constant blinking, squinting, rubbing, and pulling. The midface ages when the cheeks begin to droop, causing nasolabial folds. Nasolabial folds are the lines that run from the sides of the nose to the corners of the mouth. These folds have been treated with facial fillers. In the nose area, as a person ages, the nose elongates. Common causes of elongation are thinning of the soft tissue and loss of elasticity, which causes "drooping of the tip" and unmasking of the bone, creating a new hump. In the lower face area, as the face ages, facial tissues descend. This results in the so-called "laugh lines".

Folds and lines in this area have been treated with facial fillers. Further down on the face, the corners of the mouth may droop and descent of the jowls can create folds often referred to as "marionette" lines. Furthermore, jowls form when the cheeks sag around a fixed point along the jaw where the facial muscles attach to the jawbone. The facial muscles continue down into the neck as a sheet called the platysma muscle. This muscle often gaps in the center of the neck, creating two bands.

Various injectables have been used for restoring tissue loss in the face. In the past, injectable collagen has been used as a soft-tissue filler to fill wrinkles, lines and scars on the face. Collagen is a naturally occurring protein that supports various parts of the body including skin, tendons and ligaments. Fat injections have been used for years to add volume, fill wrinkles, lines and enhance the lips. Fat injections involve taking fat from one part of the patient's body (abdomen, thighs or buttocks) and injecting it beneath the facial skin.

Hyaluronic acid (HA), now one of the most commonly used components of cosmetic dermal fillers, is introduced into aging skin to add volume and minimize wrinkles and lines. Hyaluronic acid is a naturally occurring, water soluble polysaccharide, specifically a glycosaminoglycan, which is a major component of the extra-cellular matrix and is widely distributed in animal tissues. The identical structure of hyaluronic acid in all species and tissues makes this polysaccharide an ideal substance for use as a bio-material in health and medicine. Hyaluronic acid is present in many places in the human body. It gives volume to the skin, shape to the eyes and elasticity to the joints.

Different from animal derived hyaluronic acid, non-animal derived hyaluronic acid is free from animal proteins. This limits the risk of animal based disease transmissions or development of allergic reactions to animal proteins.

EP 2 637 710 B1

HA, also known as hyaluronan, has excellent biocompatibility and, unlike collagen, does not require any skin testing before implantation. In addition, HA has the ability to bind to large amounts of water, making it an excellent volumizer of soft tissues.

The development of HA-based fillers which exhibit ideal *in vivo* properties as well as ideal surgical usability has proven difficult. For example, HA-based fillers that exhibit desirable stability properties *in vivo*, can be so highly viscous that injection through fine gauge needles is difficult. Conversely, HA-based fillers that are relatively easily injected through fine gauge needles often have relatively inferior stability properties *in vivo.*

One method to overcome this problem is to have an adequate design of crosslinked HA-based fillers. Crosslinked HA is formed by reacting uncrosslinked HA with a crosslinking agent under suitable reaction conditions.

It is generally accepted that HA-based dermal fillers having a high viscosity, for example, those that are highly crosslinked and/or made of high molecular weight HA and/or having a high HA concentration tend to last longer in the body. Conversely, it is generally accepted that HA-based dermal fillers having a low viscosity, for example, those that are more lightly crosslinked and/or made up of low molecular weight HA and/or have a low HA concentration, may have a shorter duration in the body. Naturally, injection of a high viscosity material through a needle is relatively more difficult, and generally requires a smaller gauge needle (for instance, 21 G, 23G vs 27G, 30G) than injection of a relatively low viscosity material. It has proven difficult to develop an HA based composition that is both easy to inject through a high gauge needle (i.e. thin needle) and which has extended duration in the body. Surprisingly, many of the long lasting, highly injectable compositions of the present invention include a high percentage of relatively low molecular weight HA at relatively low concentrations.

The present invention addresses these and other issues by providing an injectable HA based dermal filler that has enhanced longevity and is extrudable through a fine needle, thus being more comfortable for the patient during injection and requiring fewer repeated visits to the physician.

US-A-2010/0255068 discloses a method for producing a soft tissue filler comprising the steps of preparing a crosslinked hydrogel material such as a crosslinked hyaluronic acid-based hydrogel, passing the crosslinked hydrogel material through a mesh once only to form hydrogel strands, and packaging the hydrogel strands.

## Summary

The present invention relates to soft tissue fillers, for example, dermal and subdermal fillers, based on hyaluronic acid (HA) and pharmaceutically acceptable salts of HA, for example, sodium hyaluronate (NaHA). In some embodiments of the invention, HA-based compositions are provided which include a therapeutically effective amount of at least one anesthetic agent, for example, but not limited to, lidocaine.

The present HA-based compositions are relatively highly viscous at rest but with low viscosity under high shear rate, thus facilitating injection even through a fine (i.e. high gauge, e.g. 27G, 30G, 31 G, 33G) needle, and have enhanced longevity in the body. The compositions may last up to four months, six months, 12 months or longer, depending on various factors such as where the compositions are introduced in the body. Methods for preparing such HA-based compositions are also provided as well as products made by such methods.

A first aspect of the invention is a soft tissue filler composition comprising:

   (1) a crosslinked hyaluronic acid (HA) component comprising a crosslinked mixture of (i) a first, low molecular weight HA material having an average molecular weight of between 0.20 MDa and 0.99 MDa, and (ii) a second, high molecular weight HA material having an average molecular weight of between 1.0 MDa and 4.0 MDa; wherein:

      the average molecular weight of the second, high molecular weight HA material is at least twice that of the first, low molecular weight HA material;
      the HA materials are hyaluronic acid or one or more salts thereof;
      the crosslinked HA component has a HA material concentration of between 15.0 mg/g and 20.0 mg/g;
      the crosslinked mixture contains more than 50% w/w of the first, low molecular weight HA material; and
      the crosslinking is achieved by the use of a crosslinking agent; and

      (2) an uncrosslinked HA solution which comprises between 27 mg/g and 33 mg/g of uncrosslinked HA and/or a salt thereof having an average molecular weight of between 1.0 MDa and 4.0 MDa, and which is present in the composition in an amount of at least 0.5% w/w and less than 5.0% w/w;

wherein each average molecular weight (MW) is calculated using the following equation:

$$MW = [\eta/(9.78 \times 10^{-5})]^{1.45}$$

wherein $\eta$ is the intrinsic viscosity in m$^3$/kg of the HA material, as measured using the method defined in the European Pharmacopoeia (Ph.Eur.), monograph no. 1472, 01/2009.

The crosslinked HA component includes more than 50%, for example, at least 70% or about 90% by weight of the first, low molecular weight HA.

The uncrosslinked HA component is a relatively high molecular weight HA material and may be present in the composition in an amount of at least 0.5% and less than about 2.0% or less than about 1.0%, or about 0.95% w/w.

The crosslinking agent may be any suitable crosslinking agent, but in a particular embodiment, the crosslinking agent is selected from the group consisting of 1,4-butanediol diglycidyl ether (BDDE), 1,4-bis(2,3-epoxypropoxy)butane, 1,4-bisglycidyloxybutane, 1,2-bis(2,3-epoxypropoxy)ethylene and 1-(2,3-epoxypropyl)-2,3-epoxycyclohexane, and 1,4-butanediol diglycidyl ether.

In some embodiments, the composition further comprises at least one active agent, for example, an anesthetic agent combined with said crosslinked HA component.

In certain specific embodiments, the crosslinked HA component has a total HA concentration of about 15.0 mg/g, about 17.0 mg/g, about 17.5 mg/g, or about 20.0 mg/g.

Another aspect of the invention is a method of making a soft tissue filler composition comprising the steps of:

preparing a crosslinked HA gel comprising a crosslinked mixture of (i) a first, low molecular weight HA material having an average molecular weight of at least 0.2 MDa and less than 1.0 MDa, and (ii) a second, high molecular weight HA material having an average molecular weight of between 1.0 MDa and 4.0 MDa, wherein the average molecular weight of the second, high molecular weight HA material is at least twice that of the first, low molecular weight material, the mixture comprises more than 50% w/w of the first, low molecular weight HA material, and the HA materials are hyaluronic acid or one or more salts thereof;
adjusting the HA material concentration in the crosslinked HA gel to between 15.0 mg/g and 20.0 mg/g;
preparing a solution comprising uncrosslinked HA and/or a salt thereof having an average molecular weight of between 1.0 MDa and 4.0 MDa, the uncrosslinked HA and/or a salt thereof being contained in the solution at a concentration of between 27 mg/g and 33 mg/g; and
combining the gel with the solution to form a composition containing the solution in an amount of at least 0.5% w/w and less than 5.0% w/w;
wherein each average molecular weight (MW) is calculated using the following equation:

$$MW = [\eta/(9.78 \times 10^{-5})]^{1.45}$$

wherein $\eta$ is the intrinsic viscosity in m$^3$/kg of the HA material, as measured using the method defined in the European Pharmacopoeia (Ph.Eur.), monograph no. 1472, 01/2009.

The composition may include at least one anesthetic agent combined with said crosslinked HA component.

In embodiments of the invention including an anesthetic agent, the agent may comprise lidocaine. In a further embodiment, the amount of the anesthetic agent is present at a concentration between about 0.1% and about 5.0% by weight of the composition. In still another embodiment, the anesthetic agent is present at a concentration between about 0.2% and about 1.0% by weight of the composition. In one embodiment, the anesthetic agent is lidocaine and is present at a concentration of about 0.3% by weight of the composition.

In yet another embodiment, the composition has a complex viscosity of between about 50 Pa*s and about 450 Pa*s, for example, when measured at about 5 Hz with a rheometer using a cone/plate geometry (4 cm/2°) at 25°C.

In one embodiment, the HA component is a gel, for example, a cohesive, hydrated gel.

In another embodiment, the composition is sterilized, for example, by autoclaving, to form a sterilized composition, wherein the sterilized composition is stable at ambient temperature for at least about 12 months, at least 18 months, at least about 24 months or more.

**Definitions**

Certain terms as used in the specification are intended to refer to the following definitions, as detailed below. Where the definition of terms departs from the commonly used meaning of the term, applicant intends to utilize the definitions provided below, unless specifically indicated.

Autoclave stable or stable to autoclaving as used herein describes a product or composition that is resistant to degradation such that the product or composition maintains at least one, and preferably all, of the following aspects after effective autoclave sterilization: transparent appearance, pH, extrusion force and/or rheological characteristics, hyaluronic acid (HA) concentration, sterility, osmolarity, and lidocaine concentration.

The term "about" in the context of numerical values means that specific values may be modified by +/- 10%. As regards endpoints of ranges, the modifier "about" means that the lower endpoint may be reduced by 10% and the upper endpoint increased by 10%. It is also contemplated that each numerical value or range disclosed in this application can be absolute, i.e. that the modifier "about" can be deleted.

All numbers herein expressing "molecular weight" of HA are to be understood as indicating the average molecular weight (Mw) in Daltons.

The molecular weight of HA is calculated from an intrinsic viscosity measurement using the following Mark Houwink relation:

$$\text{Intrinsic Viscosity } (m^3/kg) = 9.78 \times 10^{-5} \times Mw^{0.690}$$

The intrinsic viscosity is measured according to the procedure defined in the European Pharmacopoeia (HA monograph N°1472, 01/2009).

High molecular weight HA as used herein describes a HA material having a molecular weight in the range of 1.0 million Daltons (mw $\geq 10^6$ Da or 1 MDa) to 4.0 MDa. For example, the high molecular weight HA in the present compositions may have a molecular weight in the range about 1.5 MDa to about 3.0 MDa, or the high molecular weight HA may have a molecular weight of about 2.0 MDa. In another example, the high molecular weight HA may have a molecular weight of about 3.0 MDa.

Low molecular weight HA as used herein describes a HA material having a molecular weight of at least 200,000 Da (0.2 MDa) to less than 1.0 MDa, for example, between about 300,000 Da (0.3 M Da) to about 750,000 Da. (0.75 MDa), up to but not exceeding 0.99 MDa.

Preferably, there is no overlap between the molecular weight distribution of the low and high molecular weight HA materials.

Preferably, the mixture of the low molecular weight HA and high molecular weight HA has a bimodal molecular weight distribution. The mixture may also have a multi-modal distribution.

The high molecular weight HA material has an average molecular weight at least twice that of the low molecular weight HA material. For example, the composition may include a low molecular weight HA material having an average molecular weight of about 500,000 Da, and a high molecular weight HA material having an average molecular weight of about, or at least about, 1.0 MDa.

In another example, a composition in accordance with the invention may include a low molecular weight HA material having an average molecular weight of about 800,000 Da, and a high molecular weight HA material having an average molecular weight of about, or at least about, 1.6 MDa.

In a similar aspect of the invention, methods for making a HA based composition are provided which generally include the steps of selecting a low molecular weight HA material and selecting a high molecular weight HA material having a molecular weight at least twice as high as the molecular weight of the low molecular weight material, combining these high and low molecular weight materials, and crosslinking these combined materials with a suitable crosslinking agent.

Degree of crosslinking as used herein refers to the intermolecular junctions joining the individual HA polymer molecules, or monomer chains, into a permanent structure, or as disclosed herein the soft tissue filler composition. Moreover, degree of crosslinking for purposes of the present disclosure is further defined as the percent weight ratio of the crosslinking agent to HA-monomeric units within the crosslinked portion of the HA based composition. It is measured by the weight ratio of crosslinker to HA monomers (crosslinker: HA monomers).

Cohesive as used herein is the ability of a HA-based composition to retain its shape and resist compression. In some embodiments of the invention, cohesiveness includes the ability of the gel to absorb at least one time its weight of water without breaking into small pieces of gel. Cohesiveness is affected by, among other factors, the molecular weight ratio of the initial uncrosslinked HA, the degree of crosslinking, and the amount of residual uncrosslinked HA following crosslinking or added to the composition.

**Detailed Description**

The present disclosure generally relates to soft tissue fillers, for example, injectable dermal and subdermal fillers, based on hyaluronic acids (HA) and pharmaceutically acceptable salts of HA, for example, sodium hyaluronate (NaHA). In one aspect, HA-based compositions described herein include a therapeutically effective amount of at least one anesthetic agent, for example, lidocaine. Methods of making these compositions are also provided.

In one aspect of the invention, the compositions maintain at least one of, or all of, the following aspects after effective autoclave sterilization and/or prolonged storage: transparent appearance, pH for use in a patient, extrusion force and/or rheological characteristics, HA concentration, sterility, osmolarity, and lidocaine concentration. Methods or processes of preparing such HA-based compositions are also provided as well as products made by such methods or processes.

As used herein, hyaluronic acid (HA) can refer to any of its hyaluronate salts, and includes, but is not limited to, sodium hyaluronate (NaHA), potassium hyaluronate, magnesium hyaluronate, calcium hyaluronate, and combinations thereof. Both HA and pharmaceutically acceptable salts thereof can be used in this invention.

Generally, the concentration of HA in the compositions described herein is preferably at least 10 mg/mL and up to about 40 mg/mL.

In certain embodiments, the HA concentration in the crosslinked component of the present compositions is about 18.0 mg/g, about 17.0 mg/g, about 16.0 mg/g, or about 15.0 mg/g. In one embodiment, the HA concentration in the crosslinked component is about 17.5 mg/g. In another embodiment the HA concentration in the crosslinked component is 15.0 mg/g.

In addition, in embodiments with anesthetics, the concentration of one or more anesthetics is in an amount effective to mitigate pain experienced upon injection of the composition. The at least one local anesthetic can be selected from the group of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dycyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, psuedococaine, pyrrocaine, ropivacaine, salicyl alcohol, tetracaine, tolycaine, trimecaine, zolamine, and salts thereof. In one embodiment, the at least one anesthetic agent is lidocaine, such as in the form of lidocaine HCl. The compositions described herein may have a lidocaine concentration of between about 0.1% and about 5% by weight of the composition, for example, about 0.2% to about 1.0% by weight of the composition. In one embodiment, the composition has a lidocaine concentration of about 0.3% by weight (w/w %) of the composition. The concentration of lidocaine in the compositions described herein can be therapeutically effective meaning the concentration is adequate to provide a therapeutic benefit without inflicting harm to the patient.

Further, a method of preparing a HA-based composition is provided wherein the method comprises providing a precursor composition including a substantially pH neutral, crosslinked HA-based gel and adjusting the pH of the gel to a pH of greater than about 7.2, for example, about 7.5 to about 8.0. The method further comprises the step of combining a solution containing lidocaine, for example in the form of lidocaine HCl, with the slightly alkaline gel after the pH has been so adjusted and obtaining a HA-based composition including lidocaine that is stable to autoclaving.

Another method of preparing a HA-based composition containing an effective amount of lidocaine, generally comprises the steps of: providing purified NaHA material, for example, in the form of fibers; hydrating the material; and crosslinking the hydrated material with a suitable crosslinking agent to form a crosslinked HA-based gel. The method further comprises the steps of neutralizing and swelling the gel, and adding to the gel a solution containing lidocaine, preferably an acidic salt of lidocaine chlorhydrate, to form a HA/lidocaine gel. Further still, the method further comprises homogenizing the HA/lidocaine gel, and adding a small amount of an uncrosslinked HA solution. The composition is then packaged in syringes for dispensing. The syringes are then sterilized by autoclaving at an effective temperature and pressure. In accordance with the present description, the packaged and sterilized HA/lidocaine gels exhibit enhanced stability relative to HA-based compositions including lidocaine which are made using conventional methods.

The present products and compositions are considered to be sterile when exposed to temperatures of at least about 120°C to about 130°C and/or pressures of at least about 83 kPa (12 pounds per square inch (PSI)) to about 138 kPa (20 PSI) during autoclaving for a period of at least about 1 minute to about 15 minutes.

The present products and compositions also remain stable when stored for long periods of time at room temperature. Preferably, the present compositions remain stable for a period of at least about two months, or at least about six months, or at least about 9 months, or at least about 12 months, or at least about 24 months or at least about 36 months, at temperatures of at least about 25°C. In a specific embodiment, the compositions are stable at a temperature up to about 45°C for a period of at least two months.

The manufacturing process includes, in one embodiment, the initial step of providing raw HA material in the form of dry HA fibers or powder. The raw HA material may be HA, its salts and/or mixtures thereof. In a preferred embodiment, the HA material comprises fibers or powder of NaHA, for example, bacterial-sourced NaHA fibers. In some aspects of the present description, the HA material may be animal derived. The HA material may be a combination of raw materials including HA and at least one other polysaccharide, for example, glycosaminoglycan (GAG).

In one aspect of the invention, the HA material in the compositions nearly entirely comprises or consists of low molecular weight HA. In some embodiments, nearly 100% of the HA material in the present compositions may be low molecular weight HA as defined above. In other embodiments, at least 50%, at least about 70%, or at least about 90% of the HA material in the compositions is low molecular weight HA as defined above, with the remaining portion of HA being high molecular weight HA.

It will be appreciated by those of ordinary skill in the art that the selection of high and low molecular weight HA material and their relative percentages or ratios is dependent upon the desired characteristics, for example, extrusion force, elastic modulus, viscous modulus and phase angle expressed as the ratio of viscous modulus to elastic modulus, and cohesivity of the final HA-based product. For additional information that may be helpful in understanding this and other aspects of the present disclosure, see Lebreton, U.S. Patent Application Publication No. 2006/0194758.

In one embodiment, the pure, dry NaHA fibers are hydrated in an alkaline solution to produce an uncrosslinked NaHA gel. Any suitable alkaline solution may be used to hydrate the NaHA in this step, for example, but not limited to aqueous solutions containing sodium hydroxide (NaOH), potassium hydroxide (KOH), sodium bicarbonate ($NaHCO_3$), lithium hydroxide (LiOH), and the like. In another embodiment, the suitable alkaline solution is an aqueous solution containing NaOH. The resulting alkaline gel will have a pH above 7.5. The pH of the resulting alkaline gel can have a pH greater than 9, greater than 10, greater than 12, or greater than 13.

The next step in the manufacturing process involves the step of crosslinking the hydrated, alkaline NaHA gel with a suitable crosslinking agent. The crosslinking agent may be any agent known to be suitable for crosslinking polysaccharides and their derivatives via their hydroxyl groups. Suitable crosslinking agents include but are not limited to, 1,4-butanediol diglycidyl ether (or 1,4-bis(2,3-epoxypropoxy)butane or 1,4-bisglycidyloxybutane, all of which are commonly known as BDDE), 1,2-bis(2,3-epoxypropoxy)ethylene and 1-(2,3-epoxypropyl)-2,3-epoxycyclohexane. The use of more than one crosslinking agent or a different crosslinking agent is not excluded from the scope of the present disclosure. In one aspect of the present disclosure, the HA gels described herein are crosslinked using BDDE.

The step of crosslinking may be carried out using any means known to those of ordinary skill in the art. Those skilled in the art appreciate how to optimize conditions of crosslinking according to the nature of the HA, and how to carry out crosslinking to an optimized degree.

In another embodiment, the crosslinking of the HA is accomplished during hydration of the HA fibers, by hydrating the combined high and low molecular weight fibers in an alkaline solution containing a crosslinking agent, for example, BDDE.

The degree of crosslinking in the HA component of the present compositions is at least about 1% and is up to about 20% BDDE/HA, w/w, for example, between about 4% and about 12%, for example, about 10%, about 8%, about 6%, about 5%, or about 4%.

The hydrated crosslinked, HA gels may be swollen to obtain the desired HA concentration. This step can be accomplished by neutralizing the crosslinked, hydrated HA gel, for example by adding an aqueous solution containing an acid, such as HCl. The gels are then swollen in a phosphate buffered saline (PBS) solution for a sufficient time and at a low temperature.

The gels may now be purified by conventional means such as, dialysis against a phosphate buffer, or alcohol precipitation, to recover the crosslinked material, to stabilize the pH of the material and to remove any un-reacted crosslinking agent. Additional water or a slightly alkaline aqueous solution can be added to bring the concentration of the HA in the composition to a desired concentration. In some embodiments, the HA concentration of the crosslinked component of the compositions is adjusted to between about 15 mg/g and about 20 mg/g. For example, the HA concentration of the crosslinked portion of the compositions may be adjusted to yield an HA concentration of about 15 mg/g, about 17 mg/g or about 20 mg/g.

The pH of the purified, substantially pH neutral, crosslinked HA gels are preferably adjusted to cause the gel to become slightly alkaline such that the gels have a pH of greater than about 7.2, for example, about 7.5 to about 8.0. This step may be accomplished by any suitable means, for example, by adding a suitable amount of dilute NaOH, KOH, $NaHCO_3$ or LiOH, to the gels or any other alkaline molecule, solution and/or buffering composition know by one skilled in the art.

In some embodiments, an effective amount of a local anesthetic, for example, lidocaine, such as lidocaine HCl, is then added to the purified NaHA gels. For example, in some embodiments, the lidocaine HCl is provided in a powder form which is solubilized using water for injection (WFI). The gels are kept neutral with a buffer or by adjustment with diluted NaOH in order that the final HA/lidocaine composition will have a desired, substantially neutral pH. Preferably, the final HA-based filler compositions including lidocaine will have a lidocaine concentration of between about 0.1 % and about 5%, for example, about 2% by weight of the composition, or in another example about 0.3%.

After the addition of the lidocaine HCl, or alternatively, during the addition of the lidocaine HCl, the HA/lidocaine gels, or compositions, are preferably homogenized to create highly homogenous HA/lidocaine gels having a desired consistency and stability. Preferably, the homogenization step comprises mixing, stirring, or beating the gels with a controlled shearing force obtaining substantially homogenous mixtures.

The crosslinked HA/lidocaine compositions described herein display a viscosity which is dependent on the composition's properties and the presence of at least one anesthetic agent. The viscosity of the HA/lidocaine composition can be from about 50 Pa·s to about 450 Pa·s. In other embodiments, the viscosity can be from about 100 Pa·s to about 400 Pa·s, or from about 150 Pa·s to about 350 Pa·s.

After optionally homogenizing the crosslinked HA composition, an amount of uncrosslinked HA solution is added to the composition.

The added uncrosslinked HA solution has a HA concentration of between 27 mg/g and 33 mg/g. The HA in the uncrosslinked solution is a high molecular weight HA, having a molecular weight of at least 1.0 MDa up to 4.0 MDa, for example, a molecular weight of about 1.2 MDa, about 1.4 MDa, about 1.6 MDa, about 1.8 MDa, about 2.0 MDa, about 2.2 MDa about 2.4 MDa, about 2.6 MDa, about 2.8 MDa, about 3.0 MDa, about 3.2 MDa, about 3.4 MD, about 3.6 MDa, or about 3.8 The uncrosslinked HA solution is added to the crosslinked HA component to produce a composition containing the uncrosslinked HA solution in an amount of at least 0.5% and less than 5% w/w.

After adding the uncrosslinked HA gel to the crosslinked HA gel, the compositions are introduced into syringes and sterilized. Syringes useful according to the present description include any syringe known in the art capable of delivering viscous dermafiller compositions. The syringes generally have an internal volume of about 0.4 mL to about 3 mL, more preferably between about 0.5 mL and about 1.5 mL or between about 0.8 mL and about 2.5 mL. This internal volume is associated with an internal diameter of the syringe which plays a key role in the extrusion force needed to inject high viscosity dermal filler compositions. The internal diameters are generally about 4 mm to about 9 mm, more preferably from about 4.5 mm to about 6.5 mm or from about 4.5 mm to about 8.8 mm. Further, the extrusion force needed to deliver the HA compositions from the syringe is dependent on the needle gauge. The gauges of needles used generally include gauges between about 18G and about 40G, more preferably about 25G to about 33G, or from about 25G to about 30G. A person of ordinary skill in the art can determine the correct syringe dimensions and needle gauge required to arrive at a particular extrusion force requirement.

The extrusion forces displayed by the HA compositions described herein using the needle dimensions described above are at injection speeds that are comfortable to a patient. Comfortable to a patient is used to define a rate of injection that does not injure or cause excess pain to a patient upon injection to the soft tissue. One skilled in the art will appreciate that comfortable as used herein includes not only patient comfort, but also comfort and ability of the physician or medical technician injecting the HA compositions. Although certain extrusion forces may be achievable with the HA compositions of the present description, one skilled in the art understands that high extrusion forces can lead to lack of control during injection and that such lack of control may result in additional pain to the patient. Extrusion forces of the present HA/lidocaine compositions can be from about 5 N to about 20 N, or more preferably from about 8 N to about 15 N, when extruded at 13.5 mm/min in a 1.0 mL syringe with a standard needle of 30G$^{1/2}$.

Sterilization, as used herein comprises any method known in the art to effectively kill or eliminate transmissible agents, preferably without substantially altering of degrading the HA/lidocaine compositions.

One preferable method of sterilization of the filled syringes is by autoclave. Autoclaving can be accomplished by applying a mixture of heat, pressure and moisture to a sample in need of sterilization. Many different sterilization temperatures, pressures and cycle times can be used for this step. For example, the filled syringes may be sterilized at a temperature of at least about 120°C to about 130°C or greater. Moisture may or may not be utilized. The pressure applied is in some embodiments depending on the temperature used in the sterilization process. The sterilization cycle may be at least about 1 minute to about 20 minutes or more.

Another method of sterilization incorporates the use of a gaseous species which is known to kill or eliminate transmissible agents. Preferably, ethylene oxide is used as the sterilization gas and is known in the art to be useful in sterilizing medical devices and products.

A further method of sterilization incorporates the use of an irradiation source which is known in the art to kill or eliminate transmissible agents. A beam of irradiation is targeted at the syringe containing the HA composition, and the wavelength of energy kills or eliminates the unwanted transmissible agents. Preferable energy useful include, but is not limited to ultraviolet (UV) light, gamma irradiation, visible light, microwaves, or any other wavelength or band of wavelengths which kills or eliminates the unwanted transmissible agents, preferably without substantially altering of degrading the HA composition.

**EXAMPLE 1 (reference example)** <u>Manufacture of a Low Molecular Weight Soft Filler including Lidocaine</u>

90% of NaHA fibers or powder having a low molecular weight and 10% of NaHA fibers or powder having a high molecular weight, (ratio of high molecular weight to low molecular weight of 2:1) are hydrated in an alkaline solution, for example, an aqueous solution containing NaOH. The mixture is mixed at ambient temperature, about 23°C, to form a substantially homogenous, alkaline HA gel.

A crosslinking agent, BDDE, is diluted in an aqueous solution and added to the alkaline HA gel. The mixture is homogenized for several minutes.

The resulting crosslinked HA gel mixture is then heated at about 50°C for about 3 hours. The material is now a highly crosslinked HA/BDDE gel (aspect = solid gel). This crosslinked gel is then neutralized with a suitable acidic solution. The neutralized HA gel is then swollen in a phosphate buffer at a cold temperature, for example a temperature of about 5°C, to obtain a highly cohesive HA gel. In this specific example, the phosphate buffered saline solution contains water-for-injection (WFI), disodium hydrogen phosphate, and sodium dihydrogen phosphate. When neutralized and swollen, the water absorbed by the crosslinked HA component is in a weight ratio of at least 1:1, and without the gel breaking into pieces.

The swollen HA gel is then mechanical stirred and filled into dialysis membranes and dialyzed against a phosphate buffer. The HA gel is filled into dialysis membranes and dialyzed against a phosphate buffer for up to several days with regular changes of the bath, in order to remove the un-reacted crosslinker, to stabilize the pH close to neutrality (pH=7.2) and to ensure proper osmolarity of the HA gel. The osmolarity of the resulting HA gel is between about 200 mOsmol and about 400 mOsmol, most preferably about 300 mOsmol.

After dialysis, the resulting HA gel has a substantially neutral pH, preferably about 7.2.

Lidocaine chlorhydrate (lidocaine HCl) in powder form is first solubilized in WFI and filtered through a 0.2 $\mu$m filter. Dilute NaOH solution is added to the HA gel in order to reach a slightly basic pH (for example, a pH of between about 7.5 and about 8). The lidocaine HCl solution is then added to the slightly basic gel to reach a final desired concentration, for example, a concentration of about 0.3% (w/w). The resulting pH of the HA/lidocaine mixture is then about 7 and the HA concentration is about 24 mg/mL. Mechanical mixing is performed in order to obtain a proper homogeneity in a standard reactor equipped with an appropriate blender mechanism.

An amount of uncrosslinked HA gel is added to the HA/lidocaine gel mixture. Specifically, high molecular weight HA fibers are swollen in a phosphate buffer solution, in order to obtain a homogeneous viscoelastic gel. This uncrosslinked HA gel is then added to the crosslinked HA/lidocaine gel (for example, at about 1%, w/w). The resulting gel is then filled into Ready-to-Fill sterile syringes and autoclaved at sufficient temperatures and pressures for sterilization for at least about 1 minute.

After autoclaving, the final HA/lidocaine product is packaged and distributed to physicians. The autoclaved HA/lidocaine product has a viscosity, cohesivity, and extrusion force that are acceptable. No degradation of the HA/lidocaine gel product is found during testing of the product after the product has spent several months in storage.

## EXAMPLE 2

### Manufacture of a Low Molecular Weight Soft Filler of the Invention (without Lidocaine)

0.8 g of predried fibers of sodium hyaluronate (NaHA) of having a molecular weight of about 0.2 MDa is weighed out into a first receptacle.

0.2 g of predried fibers of NaHA, of having a molecular weight of about 2.4 MDa is weighed out into another receptacle.

The two different grades of NaHA are combined.

In a separate receptacle the chosen crosslinking agent, 1,4-butanediol diglycidyl ether (BDDE), is diluted in 1% sodium hydroxide solution.

6.8 g of the previously prepared BDDE solution diluted to 1/100 are then added to the mixed NaHA fibers, still in the solid state, and the mixture is homogenized mechanically with a spatula. The mixture is mixed for two hours at about 20°C to for a substantially homogenous, alkaline HA gel.

The mixture is then placed in a warm water bath at 50°C for 2 to 3 hours, with further homogenization after 15 minutes of immersion.

The resulting crosslinked HA polymer is then immersed in a phosphate buffer (PB) to stabilize the pH.

The swollen crosslinked NaHA polymer is then purified by immersion in different baths of phosphate buffer to remove unreacted crosslinking agent and HA.

Dry HA material having a high molecular weight is hydrated in 1 liter of water to obtain an uncrosslinked HA gel. 1% of this HA gel corresponding to the amount in the final composition is mixed into the crosslinked HA gel to provide a dermal filler composition in accordance with the present invention.

The hydrogel obtained is then homogenized mechanically to ensure the final homogeneity, and packed into syringes which are sterilized in an autoclave.

The gel obtained is a long lasting injectable composition using a fine gauge needle (e.g. 30 Gauge or 33 Gauge) to improve nasolabial fold lines on the face.

Although the invention has been described and illustrated with a certain degree of particularity, it is understood that the present disclosure has been made only by way of example, and that numerous changes in the combination and arrangement of parts can be resorted to by those skilled in the art without departing from the scope of the invention, as hereinafter claimed.

**Claims**

1. A soft tissue filler composition comprising:

    (1) a crosslinked hyaluronic acid (HA) component comprising a crosslinked mixture of (i) a first, low molecular weight HA material having an average molecular weight of between 0.20 MDa and 0.99 MDa, and (ii) a second, high molecular weight HA material having an average molecular weight of between 1.0 MDa and 4.0 MDa; wherein:

      the average molecular weight of the second, high molecular weight HA material is at least twice that of the first, low molecular weight HA material;
      the HA materials are hyaluronic acid or one or more salts thereof;
      the crosslinked HA component has a HA material concentration of between 15.0 mg/g and 20.0 mg/g;
      the crosslinked mixture contains more than 50% w/w of the first, low molecular weight HA material; and
      the crosslinking is achieved by the use of a crosslinking agent; and

    (2) an uncrosslinked HA solution which comprises between 27 mg/g and 33 mg/g of uncrosslinked HA or a salt thereof having an average molecular weight of between 1.0 MDa and 4.0 MDa, and which is present in the composition in an amount of at least 0.5% w/w and less than 5.0% w/w; wherein each average molecular weight (MW) is calculated using the following equation:

$$MW = [\eta/(9.78 \times 10^{-5})]^{1.45}$$

    wherein n is the intrinsic viscosity in m$^3$/kg of the HA material, as measured using the method defined in the European Pharmacopoeia (Ph.Eur.), monograph no. 1472, 01/2009.

2. The composition of claim 1, wherein the uncrosslinked HA or a salt thereof has an average molecular weight of between 2.0 MDa and 4.0 MDa.

3. The composition of claim 1, wherein the average molecular weight of the first, low molecular weight HA material is between 0.5 MDa and 0.99 MDa.

4. The composition of claim 3, wherein the average molecular weight of the first, low molecular weight HA material is in the range of 720,000-880,000 Da.

5. The composition of claim 1, wherein the crosslinked mixture contains at least 70% w/w of the first, low molecular weight HA material.

6. The composition of claim 1, wherein the crosslinking agent is selected from 1,4-butanediol diglycidyl ether (BDDE), 1,4-bis(2,3-epoxypropoxy)butane, 1,4-bisglycidyloxybutane, 1,2-bis(2,3-epoxypropoxy)ethylene, 1-(2,3-epoxypropyl)-2,3-epoxycyclohexane and 1,4-butanediol diglycidyl ether.

7. The composition of claim 1, wherein the crosslinked HA component has a HA material concentration in the range of 13.5-16.5 mg/g.

8. The composition of claim 1, wherein the uncrosslinked HA solution is present in the composition in an amount of at least 0.5% w/w and less than 1.0% w/w.

9. The composition of claim 1, further comprising at least one anesthetic agent combined with the crosslinked HA component.

10. A method of making a soft tissue filler composition comprising the steps of:

preparing a crosslinked HA gel comprising a crosslinked mixture of (i) a first, low molecular weight HA material having an average molecular weight of at least 0.2 MDa and less than 1.0 MDa, and (ii) a second, high molecular weight HA material having an average molecular weight of between 1.0 MDa and 4.0 MDa, wherein the average molecular weight of the second, high molecular weight HA material is at least twice that of the first, low molecular weight material, the mixture comprises more than 50% w/w of the first, low molecular weight HA material, and the HA materials are hyaluronic acid or one or more salts thereof;

adjusting the HA material concentration in the crosslinked HA gel to between 15.0 mg/g and 20.0 mg/g;

preparing a solution comprising uncrosslinked HA or a salt thereof having an average molecular weight of between 1.0 MDa and 4.0 MDa, the uncrosslinked HA or a salt thereof being contained in the solution at a concentration of between 27 mg/g and 33 mg/g; and

combining the gel with the solution to form a composition containing the solution in an amount of at least 0.5% w/w and less than 5.0% w/w;

wherein each average molecular weight (MW) is calculated using the following equation:

$$MW = [\eta/(9.78 \times 10^{-5})]^{1.45}$$

wherein $\eta$ is the intrinsic viscosity in $m^3/kg$ of the HA material, as measured using the method defined in the European Pharmacopoeia (Ph.Eur.), monograph no. 1472, 01/2009.

11. The method of claim 10, wherein the crosslinked mixture comprises more than 70% w/w of the first, low molecular weight HA material.

12. The method of claim 11, wherein the crosslinked mixture comprises more than 90% w/w of the first, low molecular weight HA material.

13. The method of claim 10, further comprising the step of combining at least one anesthetic agent with the gel.

14. The method of claim 13, wherein the step of combining at least one anesthetic agent with the gel is performed prior to the step of combining the gel with the solution.


**Patentansprüche**

1. Weichgewebe-Füllstoffzusammensetzung, umfassend:

(1) eine vernetzte Hyaluronsäure (HA)-Komponente, umfassend eine vernetzte Mischung aus (i) einem ersten HA-Material mit niedrigem Molekulargewicht, das ein durchschnittliches Molekulargewicht zwischen 0,20 MDa und 0,99 MDa aufweist, und (ii) einem zweiten HA-Material mit hohem Molekulargewicht, das ein durchschnittliches Molekulargewicht zwischen 1,0 MDa und 4,0 MDa aufweist;

wobei:

das durchschnittliche Molekulargewicht des zweiten HA-Materials mit hohem Molekulargewicht mindestens das Zweifache von dem des ersten HA-Materials mit niedrigem Molekulargewicht ist;

die HA-Materialien Hyaluronsäure oder ein oder mehrere Salze davon sind;

die vernetzte HA-Komponente eine HA-Materialkonzentration zwischen 15,0 mg/g und 20,0 mg/g aufweist;

die vernetzte Mischung mehr als 50 Gew.% des ersten HA-Materials mit niedrigem Molekulargewicht enthält; und

die Vernetzung unter Verwendung eines Vernetzungsmittels erzielt ist; und

(2) eine unvernetzte HA-Lösung, die zwischen 27 mg/g und 33 mg/g einer unvernetzten HA oder eines Salzes davon umfasst, die/das ein durchschnittliches Molekulargewicht zwischen 1,0 MDa und 4,0 MDa aufweist und die/das in der Zusammensetzung in einer Menge von mindestens 0,5 Gew.% und weniger als 5,0 Gew.% vorliegt;

wobei jedes durchschnittliche Molekulargewicht (MW) unter Verwendung der folgenden Gleichung berech- net wird:

$$MW = [\eta/(9{,}78 \times 10^{-5})]^{1{,}45}$$

worin $\eta$ die intrinsische Viskosität in $m^3/kg$ des HA-Materials, gemessen unter Verwendung der Methode, wie in European Pharmacopoeia (Ph.Eur.), Monograph No. 1472, 01/2009 definiert, ist.

2. Zusammensetzung gemäß Anspruch 1, wobei die unvernetzte HA oder ein Salz davon ein durchschnittliches Molekulargewicht zwischen 2,0 MDa und 4,0 MDa aufweist.

3. Zusammensetzung gemäß Anspruch 1, wobei das durchschnittliche Molekulargewicht des ersten HA-Materials mit niedrigem Molekulargewicht zwischen 0,5 MDa und 0,99 MDa ist.

4. Zusammensetzung gemäß Anspruch 3, wobei das durchschnittliche Molekulargewicht des ersten HA-Materials mit niedrigem Molekulargewicht im Bereich von 720.000 bis 880.000 Da ist.

5. Zusammensetzung gemäß Anspruch 1, wobei die vernetzte Mischung mindestens 70 Gew.% des ersten HA-Materials mit niedrigem Molekulargewicht enthält.

6. Zusammensetzung gemäß Anspruch 1, wobei das Vernetzungsmittel aus 1,4-Butandioldiglycidylether (BDDE), 1,4-Bis(2,3-expoxypropoxy)butan, 1,4-Bisglycidyloxybutan, 1,2-Bis(2,3-epoxypropoxy)ethylen, 1-(2,3-Epoxypropyl)-2,3-epoxycyclohexan und 1,4-Butandioldiglycidylether ausgewählt ist.

7. Zusammensetzung gemäß Anspruch 1, wobei die vernetzte HA-Komponente eine HA-Materialkonzentration im Bereich von 13,5 bis 16,5 mg/g aufweist.

8. Zusammensetzung gemäß Anspruch 1, wobei die unvernetzte HA-Lösung in der Zusammensetzung in einer Menge von mindestens 0,5 Gew.% und weniger als 1,0 Gew.% vorliegt.

9. Zusammensetzung gemäß Anspruch 1, zusätzlich umfassend mindestens ein Anästhetikum, kombiniert mit der vernetzten HA-Komponente.

10. Verfahren zur Herstellung einer Weichgewebe-Füllstoffzusammensetzung, umfassend die Schritte:

Herstellen eines vernetzten HA-Gels, umfassend eine vernetzte Mischung aus (i) einem ersten HA-Material mit niedrigem Molekulargewicht, das ein durchschnittliches Molekulargewicht von mindestens 0,2 MDa und weniger als 1,0 MDa aufweist, und (ii) einem zweiten HA-Material mit hohem Molekulargewicht, das ein durchschnittliches Molekulargewicht zwischen 1,0 MDa und 4,0 MDa aufweist, wobei das durchschnittliche Molekulargewicht des zweiten HA-Materials mit hohem Molekulargewicht mindestens das Zweifache von dem des ersten Materials mit niedrigem Molekulargewicht ist, die Mischung mehr als 50 Gew.% des ersten HA-Materials mit niedrigem Molekulargewicht umfasst, und die HA-Materialien Hyaluronsäure oder ein oder mehrere Salze davon sind; Einstellen der HA-Materialkonzentration in dem vernetzten HA-Gel auf 15,0 mg/g bis 20,0 mg/g; Herstellen einer Lösung, umfassend unvernetzte HA oder ein Salz davon, die/das ein durchschnittliches Molekulargewicht zwischen 1,0 MDa und 4,0 MDa aufweist, wobei die unvernetzte HA oder ein Salz davon in einer Konzentration zwischen 27 mg/g und 33 mg/g in der Lösung enthalten ist; und Kombinieren des Gels mit der Lösung, um eine Zusammensetzung zu bilden, die die Lösung in einer Menge von mindestens 0,5 Gew.% und weniger als 5,0 Gew.% enthält; wobei jedes durchschnittliche Molekulargewicht (MW) unter Verwendung der folgenden Gleichung berechnet wird:

$$MW = [\eta/(9{,}78 \times 10^{-5})]^{1{,}45}$$

worin $\eta$ die intrinsische Viskosität in $m^3/kg$ des HA-Materials, gemessen unter Verwendung des Verfahrens, wie in European Pharmacopoeia (Ph.Eur.), Monograph No. 1472, 01/2009 definiert, ist.

11. Verfahren gemäß Anspruch 10, bei dem die vernetzte Mischung mehr als 70 Gew.% des ersten HA-Materials mit niedrigem Molekulargewicht umfasst.

**12.** Verfahren gemäß Anspruch 11, bei dem die vernetzte Mischung mehr als 90 Gew.% des ersten HA-Materials mit niedrigem Molekulargewicht umfasst.

**13.** Verfahren gemäß Anspruch 10, zusätzlich umfassend den Schritt des Kombinierens von mindestens einem Anästhetikum mit dem Gel.

**14.** Verfahren gemäß Anspruch 13, bei dem der Schritt des Kombinierens des mindestens einen Anästhetikums mit dem Gel vor dem Schritt des Kombinierens des Gels mit der Lösung durchgeführt wird.


**Revendications**

**1.** Composition de comblement de tissus mous comprenant :

(1) un composant d'acide hyaluronique (AH) réticulé comprenant un mélange réticulé de (i) un premier matériau d'AH de faible poids moléculaire ayant un poids moléculaire moyen compris entre 0,20 MDa et 0,99 MDa, et (ii) un second matériau d'AH de haut poids moléculaire ayant un poids moléculaire moyen compris entre 1,0 MDa et 4,0 MDa ;
où
le poids moléculaire moyen du second matériau d'AH de haut poids moléculaire est au moins deux fois celui du premier matériau d'AH de faible poids moléculaire;
les matériaux d'AH sont l'acide hyaluronique ou un ou plusieurs sels de ce dernier;
le composant d'AH réticulé a une concentration en matériau d'AH comprise entre 15,0 mg/g et 20,0 mg/g ;
le mélange réticulé contient plus de 50 % p/p du premier matériau d'AH de faible poids moléculaire; et
la réticulation est réalisée par l'utilisation d'un agent de réticulation ; et
(2) une solution d'AH non réticulé comprenant entre 27 mg/g et 33 mg/g d'AH non réticulé ou d'un sel de ce dernier ayant un poids moléculaire moyen compris entre 1,0 MDa et 4,0 MDa, et qui est présente dans la composition en une quantité d'au moins 0,5 % p/p et de moins de 5,0 % p/p ;
où chaque poids moléculaire (PM) moyen est calculé par l'utilisation de l'équation suivante :

$$PM = [\eta/(9,78 \times 10^{-5})]^{1,45}$$

où $\eta$ est la viscosité intrinsèque en $m^3/kg$ du matériau d'AH, mesurée par l'utilisation de la méthode définie dans la Pharmacopée Européenne (Ph.Eur.), monographie n° 1472, 01/2009.

**2.** Composition selon la revendication 1, dans laquelle l'AH non réticulé ou un sel de ce dernier a un poids moléculaire moyen compris entre 2,0 MDa et 4,0 MDa.

**3.** Composition selon la revendication 1, dans laquelle le poids moléculaire moyen du premier matériau d'AH de faible poids moléculaire est compris entre 0,50 MDa et 0,99 MDa.

**4.** Composition selon la revendication 3, dans laquelle le poids moléculaire moyen du premier matériau d'AH de faible poids moléculaire est situé dans la gamme allant de 720 000 à 880 000 Da.

**5.** Composition selon la revendication 1, dans laquelle le mélange réticulé comprend au moins 70 % p/p du premier matériau d'AH de faible poids moléculaire.

**6.** Composition selon la revendication 1, dans laquelle l'agent de réticulation est choisi dans le groupe constitué par l'éther diglycidylique de 1,4-butanediol (EDBD), le 1,4-bis(2,3-époxypropoxy)butane, le 1,4-bisglycidyloxybutane, le 1,2-bis(2,3-époxypropoxy)éthylène, le 1-(2,3-époxypropyl)-2,3-époxycyclohexane et l'éther diglycidylique de 1,4-butanediol.

**7.** Composition selon la revendication 1, dans laquelle le composant d'AH réticulé a une concentration en matériau d'AH située dans la gamme allant de 13,5 mg/g à 16,5 mg/g ;

8. Composition selon la revendication 1, dans laquelle la solution d'AH non réticulé est présente dans la composition en une quantité d'au moins 0,5 % p/p et de moins de 1,0 % p/p.

9. Composition selon la revendication 1, comprenant en outre au moins un agent anesthésique combiné avec le composant d'AH réticulé.

10. Procédé de fabrication d'une composition de comblement de tissus mous comprenant les étapes consistant à :

préparer un gel d'AH réticulé comprenant un mélange réticulé de (i) un premier matériau d'AH de faible poids moléculaire ayant un poids moléculaire moyen d'au moins 0,20 MDa et moins de 1,0 MDa, et (ii) un second matériau d'AH de haut poids moléculaire ayant un poids moléculaire moyen compris entre 1,0 MDa et 4,0 MDa, où le poids moléculaire moyen du deuxième matériau d'AH de haut poids moléculaire est au moins deux fois celui du premier matériau d'AH de faible poids moléculaire, le mélange comprenant plus de 50 % p/p du premier matériau d'AH de faible poids moléculaire, et les matériaux d'AH sont l'acide hyaluronique ou un ou plusieurs sels de ce dernier ;
ajuster la concentration du matériau d'AH dans le gel d'AH réticulé à des valeurs comprises entre 15,0 mg/g et 20,0 mg/g;
préparer une solution comprenant de l'AH non réticulé ou d'un sel de ce dernier ayant un poids moléculaire moyen compris entre 1,0 MDa et 4,0 MDa, le AH non réticulé ou un sel de ce dernier étant contenu dans la solution à une concentration comprise entre 27 mg/g et 33 mg/g ; et
combiner le gel avec la solution pour former une composition contenant la solution en une quantité d'au moins 0,5 % p/p et de moins de 5,0 % p/p ;
où chaque poids moléculaire (PM) moyen est calculé par l'utilisation de l'équation suivante :

$$PM = [\eta/(9{,}78 \times 10^{-5})]^{1{,}45}$$

où $\eta$ est la viscosité intrinsèque en m³/kg du matériau d'AH, mesurée par l'utilisation du procédé défini dans la Pharmacopée Européenne (Ph.Eur.), monographie n° 1472, 01/2009.

11. Procédé selon la revendication 10, dans lequel le mélange réticulé comprend plus de 70 % p/p du premier matériau d'AH de faible poids moléculaire.

12. Procédé selon la revendication 11, dans lequel le mélange réticulé comprend plus de 90 % p/p du premier matériau d'AH de faible poids moléculaire.

13. Procédé selon la revendication 10, comprenant en outre l'étape consistant à combiner au moins un agent anesthésique avec le gel.

14. Procédé selon la revendication 13, dans lequel l'étape consistant à combiner au moins un agent anesthésique avec le gel est mise en oeuvre préalablement à l'étape consistant à combiner le gel avec la solution.

**EP 2 637 710 B1**

## REFERENCES CITED IN THE DESCRIPTION

**Patent documents cited in the description**

- US 20100255068 A
- US 20060194758 A